# FASCICULE DE BREVET EUROPEEN

(11) **EP 1 935 443 B1**
(45) Date de publication et mention de la délivrance du brevet: **15.05.2013**
(21) Numéro de dépôt: 07019372.7
(22) Date de dépôt: 02.10.2007
(51) Int. Cl.: A61M 5/168

(54) **Tubulure de nutrition universelle à usage unique pour pompe à péristaltisme rotatif**
Universal-Einweg-Ernährungsschlauch für Peristaltik-Pumpe
Single-use universal nutrition tubing for a pump with rotary peristalsis

(30) Priorité: 21.11.2006 FR 0610189
(43) Date de publication de la demande: 25.06.2008
(73) Titulaire: Medwin France, 34240 Lamalou les Bains (FR)
(72) Inventeur: Renaux, Serge, 34600 Les Aires (FR)
(74) Mandataire: Gosse, Michel

(56) Documents cités:
- EP-A- 0 170 984
- EP-A2- 1 559 442
- FR-A1- 2 860 160
- US-A- 4 548 600
- US-A- 5 147 313

## Description

### DOMAINE DE L'INVENTION

La présente invention se rapporte à une tubulure de nutrition universelle à usage unique conçue pour être utilisée sur différents types de pompes à péristaltisme rotatif commercialisées par des fournisseurs différents sur un marché donné.

### ARRIERE PLAN TECHNOLOGIQUE

Les tubulures de nutrition connues appartenant au domaine de l'invention comprennent généralement :
a) une chambre compte goutte destinée à être placée dans un premier logement de la pompe servant de support et contenant un capteur générant une onde infrarouge orientée de manière à être occultée par les gouttes chutant par gravité dans ladite chambre qui comporte, en partie inférieure, un embout pour tubulure ;
b) un capot, obturant la partie supérieure de ladite chambre, pourvu d'une tétine dont la forme et les dimensions sont définies pour générer des gouttes aux dimensions souhaitées pour être détectées correctement par le rayonnement infrarouge lors de leur chute par gravité ; ledit capot comportant, en partie supérieure, un embout destiné à être relié à une tubulure d'amenée du nutriment ;
c) un raccord de pompe destiné à être placé dans un deuxième logement de la pompe servant de support et pourvu, en partie basse, d'un embout pour tubulure et, en partie haute, d'un embout destiné à être relié à une tubulure d'administration du nutriment au patient ;
d) un segment de tubulure reliant l'embout inférieur de la chambre compte goutte à l'embout inférieur du raccord de pompe et passant, tendu, par le rotor du moteur de la pompe péristaltique.

Elles présentent toutes les inconvénients liés au fait qu'il y a sur le marché autant de types de tubulures que de modèles de pompes appartenant à différents fournisseurs.

Par exemple, les 4 principaux fournisseurs de pompes à péristaltisme rotatif sur le marché français (Nestlé, Novartis, Nutricia, Frésénius), proposent 6 modèles spécifiques de tubulures consommables pour 6 modèles de pompes.

Ainsi, la chambre compte goutte et le raccord de pompe ont des dimensions et une forme propre à chaque type de pompe.

Par contre, leur fonctionnement est commun à toutes les pompes, à savoir : une chambre compte goutte placée dans un logement équipé d'un capteur infrarouge pour le nutriment, un moteur à péristaltisme rotatif et un raccord de pompe, le tout étant relié à une tubulure d'amenée de nutriment et tubulure d'administration du nutriment au patient.

Les nombreuses références liées au fait qu'il y a autant de types de tubulures de nutrition que de modèles de pompes, s'expliquent par l'écart dimensionnel existant pour les logements de deux composants : la chambre compte goutte et le raccord de pompe. Ces deux éléments clés de la tubulure, sont reliés entre eux par un segment qui une fois en contact avec le rotor du moteur de la pompe, assure la progression des nutriments.

Les principaux inconvénients qui en résultent se situent à plusieurs niveaux :
- d'abord pour le fabricant qui doit prévoir plusieurs types de moules et de lignes de montage ;
- ensuite pour le distributeur qui doit multiplier le nombre de références ;
- enfin pour le patient pour lequel le risque d'erreur est accru.

Le document EP-A-0 170 984 décrit une tubulure de nutrition à usage unique comprenant : une chambre compte-goutte pour pompe péristaltique et un capot obturant la partie supérieure de la chambre.

### RESUME DE L'INVENTION

L'invention vise donc à mettre en oeuvre une tubulure de nutrition nouvelle et originale qui supprime les inconvénients susmentionnés.

Un des buts de la présente invention est de fournir une tubulure unique qui s'adapte aux différents types de pompes commercialisées sur un marché donné.

Pour ce faire, la tubulure selon l'invention se caractérise essentiellement en ce que :
- le corps de la chambre compte gouttes comporte au moins deux zones jointives superposées, sensiblement tronconiques, dont le diamètre extérieur et la longueur sont définis pour permettre leur insertion dans des logements de dimensions différentes appartenant à des pompes de types différents et pour permettre au rayonnement infrarouge de chaque type de pompe d'être occulté par les gouttes à compter ;
- le corps du raccord de pompe comporte au moins deux zones jointives superposées, sensiblement tronconiques, dont le diamètre extérieur et la longueur sont définis pour permettre leur insertion dans des logements de dimensions différentes appartenant à des pompes de types différents.

Un autre but de la présente invention est d'utiliser une tétine qui comporte, dans son canal interne, des rainures longitudinales destinées à générer des gouttes aux dimensions souhaitées pour être détectées correctement par le rayonnement infrarouge appartenant aux différents types de pompes lors de leur chute par gravité.

Un autre but de la présente invention est d'utiliser un capot clipsable à l'extrémité de la chambre compte goutte afin d'éviter le collage qui est source de souillures modifiant la transparence de ladite chambre.

### PRESENTATION DES FIGURES

Les caractéristiques et les avantages de l'invention vont apparaître plus clairement à la lecture de la description détaillée qui suit d'au moins un mode de réalisation préféré de celle-ci donné à titre d'exemple non limitatif et représenté aux dessins annexés.

Sur ces dessins :

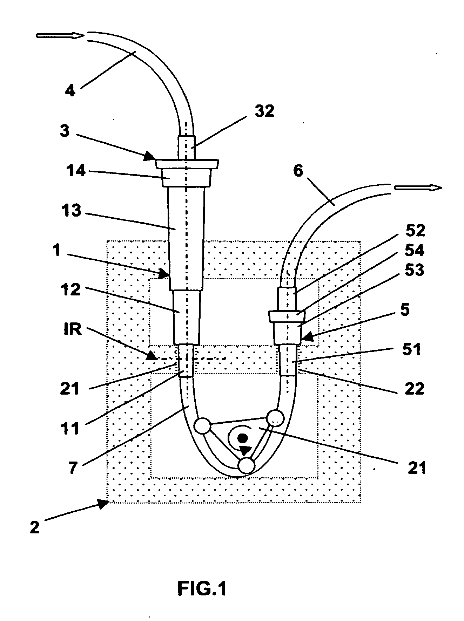

- la figure 1 représente la réalisation préférée de l'ensemble de la tubulure selon l'invention positionnée sur la pompe ;

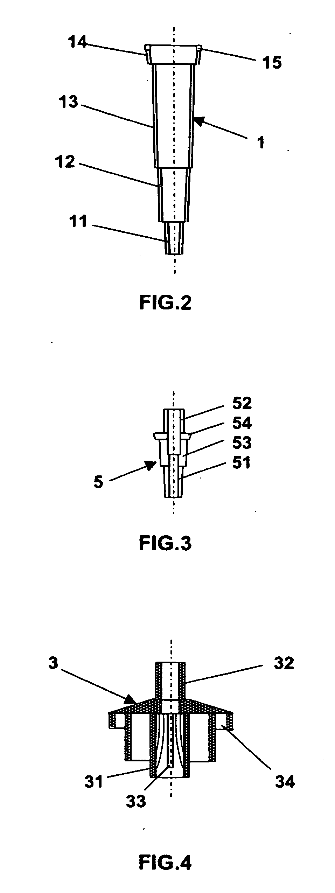

- la figure 2 représente, en coupe longitudinale, la chambre selon la figure 1 ;
- la figure 3 représente, en coupe longitudinale, le raccord de pompe selon la figure 1 ;
- la figure 4 représente, en coupe longitudinale, le capot selon la figure 1.

### DESCRIPTION DETAILLEE DE L'INVENTION

La tubulure représentée aux figures est du genre comprenant :
a) une chambre compte goutte (1) destinée à être placée dans un premier logement (21) d'une pompe péristaltique rotative (2), ledit logement contenant un capteur générant une onde infrarouge (IR) orientée de manière à être occultée par les gouttes chutant par gravité dans ladite chambre qui est pourvue, en partie inférieure, d'un embout (11) destiné à la fixation d'une tubulure (7) ;
b) un capot (3), obturant la partie supérieure de ladite chambre, pourvu d'une tétine (31) dont la forme et les dimensions sont définies pour générer des gouttes aux dimensions souhaitées pour être détectées correctement par le rayonnement infrarouge lors de leur chute par gravité, ledit capot comportant, en partie supérieure, un embout (32) destiné à la fixation d'une tubulure (4) d'amenée du nutriment ;
c) un raccord de pompe (5), destiné à être placé dans un deuxième logement (22) de la pompe (2), pourvu, en partie basse, d'un embout (51) destiné à la fixation d'une tubulure (7) et, en partie haute, d'un embout (52) destiné à la fixation d'une tubulure (6) d'administration du nutriment au patient ;
d) un segment de tubulure (7) reliant l'embout inférieur (51) de la chambre compte goutte à l'embout inférieur (11) du raccord de pompe et passant, tendu, par le rotor (21) du moteur de la pompe péristaltique (2).

Selon les caractéristiques de base de l'invention :
- le corps de la chambre compte goutte (1) comporte au moins deux zones jointives superposées (12) et (13), sensiblement tronconiques, dont le diamètre extérieur et la longueur sont définis pour permettre leur insertion dans des logements (21) de dimensions différentes appartenant à des pompes (2) de types différents et pour permettre au rayonnement infrarouge (IR) de chaque type de pompe d'être occulté par les gouttes à compter ;
- le corps du raccord de pompe (5) comporte au moins deux zones jointives superposées (53) et (54), sensiblement tronconiques, dont le diamètre extérieur et la longueur sont définis pour permettre leur insertion dans des logements (22) de dimensions différentes appartenant à des pompes de types différents ;
- la tétine (31) comporte, dans son canal interne, des rainures longitudinales (33) destinées à générer des gouttes de dimensions aptes à être détectées correctement par le rayonnement infrarouge lors de leur chute par gravité.

Selon d'autres particularités de réalisation de l'invention :
- la partie supérieure du corps de la chambre compte goutte (1) comporte une zone (14), sensiblement tronconique, servant de réducteur de section, dont l'extrémité (15) est destinée à venir se clipser dans un logement circulaire approprié (34) du capot (3) ;
- la tubulure (4) d'amenée du nutriment, réalisée en PVC, est insérée dans l'embout (32) ;
- la tubulure (6) de distribution du nutriment au patient, réalisée avantageusement en PVC, est insérée dans l'embout (52) ;
- le segment de tubulure (7), réalisé avantageusement en silicone, est emmanché sur les embouts (11) et (51) ;
- la chambre compte goutte est réalisée avantageusement en polyéthylène haute densité ou en PVC ;
- le raccord de pompe est réalisé avantageusement en ABS.

Bien entendu, l'invention n'est pas limitée aux modes de réalisation décrits et représentés pour lesquels l'homme de métier pourra prévoir d'autres variantes, en particulier dans la conception des sous-ensembles constitutifs de l'invention et la nature des matériaux utilisés.

## Revendications

1. Tubulure de nutrition à usage unique, comprenant :
a) une chambre compte goutte (1) destinée à être placée dans un premier logement (21) d'une pompe péristaltique rotative (2), ledit logement contenant un capteur générant une onde infrarouge (IR) orientée de manière à être occultée par les gouttes chutant par gravité dans ladite chambre qui est pourvue, en partie inférieure, d'un embout (11) destiné à la fixation d'une tubulure (7) ;
b) un capot (3), obturant la partie supérieure de ladite chambre, pourvu d'une tétine (31) dont la forme et les dimensions sont définies pour générer des gouttes aux dimensions souhaitées pour être détectées correctement par le rayonnement infrarouge lors de leur chute par gravité, ledit capot comportant, en partie supérieure, un embout (32) destiné à la fixation d'une tubulure (4) d'amenée du nutriment ;
c) un raccord de pompe (5), destiné à être placé dans un deuxième logement (22) de la pompe (2), pourvu, en partie basse, d'un embout (51) destiné à la fixation d'une tubulure (7) et, en partie haute, d'un embout (52) destiné à la fixation d'une tubulure (6) d'administration du nutriment au patient ;
d) un segment de tubulure (7) reliant l'embout inférieur (51) de la chambre compte goutte à l'embout inférieur (11) du raccord de pompe et passant, tendu, par le rotor (21) du moteur de la pompe péristaltique (2) ;
**caractérisée en ce que** le corps de la chambre compte goutte (1) comporte au moins deux zones jointives superposées (12) et (13), sensiblement tronconiques, dont le diamètre extérieur et la longueur sont définis pour permettre leur insertion dans des logements (21) de dimensions différentes appartenant à des pompes (2) de types différents et pour permettre au rayonnement infrarouge (IR) de chaque type de pompe d'être occulté par les gouttes à compter.

2. Tubulure de nutrition, selon la revendication 1, **caractérisée en ce que** la tétine (31) comporte, dans son canal interne, des rainures longitudinales (33) destinées à générer des gouttes de dimensions aptes à être détectées correctement par le rayonnement infrarouge lors de leur chute par gravité.

3. Tubulure de nutrition, selon la revendication 1, **caractérisée en ce que** le corps du raccord de pompe (5) comporte au moins deux zones jointives superposées (53) et (54), sensiblement tronconiques, dont le diamètre extérieur et la longueur sont définis pour permettre leur insertion dans des logements (22) de dimensions différentes appartenant à des pompes de types différents.

4. Tubulure de nutrition, selon la revendication 1, **caractérisée en ce que** la partie supérieure du corps de la chambre compte goutte (1) comporte une zone (14), sensiblement tronconique, servant de réducteur de section, dont l'extrémité (15) est destinée à venir se clipser dans un logement circulaire approprié (34) du capot (3).

## Patentansprüche

1. Einweg-Ernährungsschlauch, umfassend:
a) eine Tropfenzählerkammer (1), die dazu bestimmt ist, in eine erste Aufnahme (21) einer Schlauchquetschpumpe (2) eingesetzt zu werden, wobei die besagte Aufnahme einen Sensor umfasst, der eine Infrarotstrahlung (IR) generiert, die so ausgerichtet ist, dass sie von den Tropfen die mittels Schwerkraft in die besagte Kammer fallen, bedeckt wird, und die im unteren Abschnitt mit einem Ansatz (11) versehen ist, der für die Befestigung eines Schlauches (7) bestimmt ist;
b) eine Abdeckung (3), die den oberen Abschnitt der besagten Kammer abdeckt, und mit einer Tülle (31) versehen ist, deren Form und Abmessungen so festgelegt sind, dass sie Tropfen in gewünschten Größen generiert, um bei deren Fall mittels Schwerkraft von der Infrarotstrahlung korrekt erfasst zu werden, wobei die besagte Abdeckung in ihrem oberen Abschnitt einen Ansatz (32) umfasst, der für die Befestigung eines Schlauches (4) zur Zuführung der Nährstoffe bestimmt ist;
c) einen Pumpenanschluss (5), der dazu bestimmt ist, in einer zweiten Aufnahme (22) der Pumpe (2) eingesetzt zu werden, der im unteren Abschnitt einen Ansatz (51) umfasst, der zur Befestigung eines Schlauches (7) bestimmt ist, und der im oberen Abschnitt einen Ansatz (52) umfasst, der zur Befestigung eines Schlauches (6) zur Verabreichung der Nährstoffe an den Patienten bestimmt ist;
d) ein Schlauchsegment (7) zur Verbindung des unteren Ansatzes (51) der Tropfenzählerkammer mit dem unteren Ansatz (11) des Pumpenanschlusses, das gespannt durch den Rotor (21) des Motors der Schlauchquetschpumpe (2) führt;
**dadurch gekennzeichnet, dass** das Gehäuse der Tropfenzählerkammer (1) zumindest zwei fugendicht übereinander liegende Bereiche (12) und (13) umfasst, die im Wesentlichen kegelig sind, und deren Außendurchmesser und Länge so definiert sind, dass sie in Aufnahmen (21) mit unterschiedlichen Abmessungen eingelegt werden können, die zu Pumpen (2) unterschiedlicher Ausführung gehören, und um es der Infrarotstrahlung (IR) jedes Pumpentyps zu ermöglichen, von den zu zählenden Tropfen bedeckt zu werden.

2. Ernährungsschlauch nach Anspruch 1, **dadurch gekennzeichnet, dass** die Tülle (31) in ihrem inneren Kanal längs verlaufende Rillen (33) umfasst, die dazu bestimmt sind, Tropfen in einer Abmessung zu generieren, die bei deren Fall mittels Schwerkraft von der Infrarot-Strahlung korrekt erfasst werden können.

3. Ernährungsschlauch nach Anspruch 1, **dadurch gekennzeichnet, dass** das Gehäuse des Pumpenanschlusses (5) zumindest zwei fugendicht übereinander liegende Bereiche (53) und (54) umfasst, die im Wesentlichen kegelig sind, und deren Außendurchmesser und Länge so definiert sind, dass sie in Aufnahmen (22) mit unterschiedlichen Abmessungen eingelegt werden können, die zu Pumpen unterschiedlicher Ausführung gehören.

4. Ernährungsschlauch nach Anspruch 1, **dadurch gekennzeichnet, dass** der obere Abschnitt des Gehäuses der Tropfenzählerkammer (1) einen Bereich (14) umfasst, der im Wesentlichen kegelig ist, und als Querschnittsverringerung dient, dessen Ende (15) dazu bestimmt ist, sich in eine geeignete kreisförmige Aufnahme (34) der Abdeckung (3) einzuklinken.

## Claims

1. Single-use nutrition tubing, comprising:
a) a vented drip chamber (1) intended to be placed in a first housing (21) of a rotating peristaltic pump (2), said housing containing a sensor generating an infrared wave (IR) directed in such a way as to be occulted by the drips falling by gravity in said chamber which is provided, in the lower portion, with a tip (11) intended for the fastening of a tubing (7);
b) an enclosure (3), closing the upper portion of said chamber, provided with a teat (31) of which the shape and the dimensions are defined for generating drips with the desired dimensions in order to be correctly detected by the infrared radiation during their fall by gravity, said enclosure comprising, in the upper portion, a tip (32) intended for the fastening of a tubing (4) for supplying the nutrient;
c) a pump connector (5), intended to be placed in a second housing (22) of the pump (2), provided, in the lower portion, with a tip (51) intended for the fastening of a tubing (7) and, in the upper portion, with a tip (52) intended for the fastening of a tubing (6) for administering the nutrient to the patient;
d) a segment of tubing (7) connecting the lower tip (51) of the vented drip chamber to the lower tip (11) of the pump connector and passing, tight, through the rotor (21) of the motor of the peristaltic pump (2);
**characterised in that** the body of the vented drip chamber (1) comprises at least two superimposed joint zones (12) and (13), substantially tapered, of which the external diameter and the length are defined for permitting insertion of the zones in the housings (21) of different dimensions belonging to pumps (2) of different types and for permitting the infrared radiation (IR) of each type of pump to be occulted by the drips to be counted.

2. Nutrition tubing, according to claim 1, **characterised in that** the teat (31) comprises, in its internal channel, longitudinal grooves (33) intended to generate drips with dimensions able to be correctly detected by the infrared radiation when they fall by gravity.

3. Nutrition tubing, according to claim 1, **characterised in that** the body of the pump connector (5) comprises at least two superimposed joint zones (53) and (54), substantially tapered, of which the external diameter and the length are defined for permitting insertion of the zones in the housings (22) of different dimensions belonging to pumps of different types.

4. Nutrition tubing, according to claim 1, **characterised in that** the upper portion of the body of the vented drip chamber (1) comprises a zone (14), substantially tapered, used as a section reducer, of which the end (15) is intended to snap-fit into a suitable circular housing (34) of the enclosure (3).
